# EUROPÄISCHE PATENTANMELDUNG

(11) **EP 3 016 150 A1**
(43) Veröffentlichungstag der Anmeldung: **04.05.2016**
(21) Anmeldenummer: 14191386.3
(22) Anmeldetag: 31.10.2014
(51) Int. Cl.: H01L 31/042, H01L 31/054

(54) **Dekorativer Verbundkörper mit Solarzelle**

(71) Anmelder: D. Swarovski KG, 6112 Wattens (AT)
(72) Erfinder: Kurtze, Andreas, 6122 Fritzens (AT); Lexer, Franz, 6094 Axams (AT); Mair, Mathias, 6176 Völs (AT)
(74) Vertreter: Fabry, Bernd

(57) **Zusammenfassung**

Vorgeschlagen wird ein Energieelement, enthaltend
(a) ein facettiertes, transparentes Objekt mit konvexer Geometrie (1),
(b) eine wellenlängenselektive Schicht (3) und
(c) eine photovoltaische Zelle (2).

## Beschreibung

### GEBIET DER ERFINDUNG

Die Erfindung betrifft ein Energieelement, welches einen facettierten, transparenten Körper mit konvexer Geometrie, eine wellenlängenselektive Schicht und ein photovoltaisches Element enthält und zur Energieversorgung, unter anderem im Bereich tragbarer Elektronik, geeignet ist.

### STAND DER TECHNIK

Bisher wurden facettierte Objekte/Schmucksteine nahezu ausschließlich für rein ästhetische Zwecke in Accessoires und auf Textilien eingesetzt, hatten aber kaum funktionale Wirkung. Im Bereich tragbarer Elektronik (sogenannte "Wearable Technologies"), einem Markt mit enormen Wachstumschancen, fehlen sie, da dieser Bereich von den Anwendern mehr mit Funktionalität als mit Schmuck verbunden wird. Eine der größten Herausforderungen im Bereich der "Wearable Technologies", etwa Körpersensoren, "Smart-Watches" oder Datenbrillen, stellt die Energieversorgung dar, deren abrupter Ausfall die Geräte zu oft unerwarteten Zeitpunkten funktionsunfähig macht.

Aus der Patentanmeldung US 2013/0329402 ist die Energieversorgung über eine eingebaute Solarzelle für dekorative Elemente bekannt.

Solarzellen werden gemäß der Patentschrift US 4,173,229 auch in Armbändern und Ketten eingesetzt, um einen therapeutisch wirksamen, elektrischen Strom durch den Körper der Schmuckträger zu leiten.

Das deutsche Gebrauchsmuster DE 203 03 952 U1 schlägt die Verwendung von Solarzellen in "Alarmverschlüssen" zur Sicherung von Schmuck vor.

Bisher fehlt jegliche technische Lösung, Solarzellen dekorativ anspruchsvoll zu gestalten, so dass sie auch als Schmucksteine verarbeitet werden können. Aufgabe der vorliegenden Erfindung war es, eine Solarzelle (photovoltaische Zelle) so zu adaptieren, dass ein hoch brillantes dekoratives Energieelement erhalten wird.

### BESCHREIBUNG DER ERFINDUNG

Ein erster Gegenstand der vorliegenden Erfindung betrifft ein Energieelement, welches
(a) ein facettiertes, transparentes Objekt mit konvexer Geometrie,
(b) eine wellenlängenselektive Schicht und
(c) eine photovoltaische Zelle
enthält.

Die Komponenten des Energieelementes sind vorzugsweise mit einem Klebstoff untereinander verbunden. Ein weiterer Gegenstand der vorliegenden Erfindung ist die Verwendung des erfindungsgemäßen Elementes als Energiequelle, insbesondere in tragbaren elektronischen Geräten. Ebenso sind Gegenstände enthaltend ein erfindungsgemäßes Energieelement Gegenstand der Erfindung. Beispielweise kann das Energieelement vorteilhaft in sogenannte "Activity Tracker" eingebaut werden, die somit ebenfalls Gegenstand der Erfindung sind. Weitere Anwendungsmöglichkeiten werden nachfolgend erwähnt.

Überraschenderweise wurde gefunden, dass eine Kombination aus einem facettierten, transparenten Objekt mit konvexer Geometrie, mit einer wellenlängenselektiven Schicht und einer photovoltaischen Zelle sich als Energiequelle für die verschiedensten Zwecke eignet. Im Sinne der Erfindung werden die Begriffe photovoltaische Zelle, photovoltaisches (PV) Element und Solarzelle synonym benutzt. Die erfindungsgemäßen Verbundkörper haben nicht nur verbesserte energieversorgende Eigenschaften, sie sind gleichzeitig Schmucksteine mit hoher Brillanz. Die Erfindung stellt somit energieliefernde, funktionale Schmucksteine bereit, die nicht nur brillant sind, sondern einen Energieertrag liefern, der höher als bei konventionellen, flachen Solarzellen ist.

Die Kombination einer photovoltaischen Zelle mit einem facettierten, transparenten Objekt mit konvexer Geometrie und einer wellenlängenselektiven Schicht, liefert vielfältige Einsatzmöglichkeiten im Design- und Technologiebereich, sowohl als Energiequelle als auch als Schmuckstein. Nachfolgend wird das facettierte, transparente Objekt mit konvexer Geometrie auch als "optisches Element" bezeichnet. Die Energie-Elemente sind hoch-brillant und ermöglichen somit nicht nur den Einsatz als Energiequelle, sondern auch als sehr dekorativer Schmuckstein. Unter Transparenz wird die Fähigkeit von Materie, elektromagnetische Wellen hindurchzulassen (Transmission) verstanden. Ist ein Material für einfallende elektromagnetische Strahlung (Photonen) eines mehr oder weniger breiten Frequenzspektrums transparent, kann diese das Material nahezu vollständig durchdringen, wird also kaum reflektiert und kaum absorbiert. Erfindungsgemäß wird unter Transparenz eine Transmission von wenigstens 80 % des einfallenden Lichtes verstanden, vorzugsweise mehr als 90 %.

Mögliche Aufbauweisen des Energieelements (Verbundkörper) sind in den **Abbildungen (1a) bis (1c)** dargestellt, wobei die Bezugszeichen folgende Bedeutung haben:
(1) facettiertes, transparentes Objekt mit konvexer Geometrie;
(2) photovoltaische Zelle (Solarzelle);
(3) wellenlängenselektive Beschichtung;
(4) wellenlängenselektive Folie;
(5) Klebstoff;
(6) unterschiedlicher Aufbau bezüglich der Lage der wellenlängenselektiven Schicht;
(7) Energieelement.

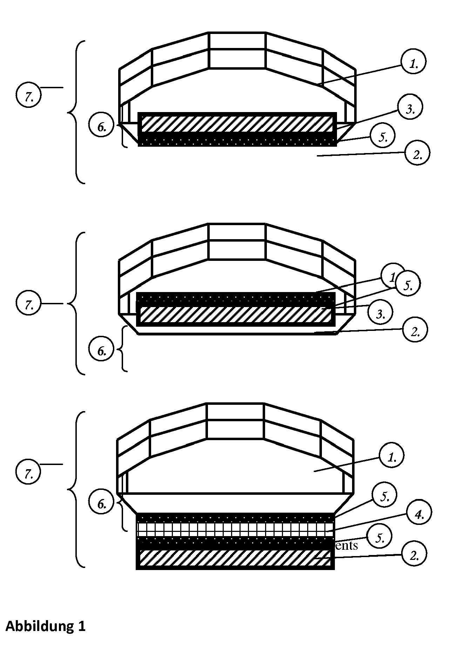

Die wellenlängenselektive Beschichtung (*vide infra*) kann sich in einer erfindungsgemäßen Ausführungsform direkt auf der der Facettierung gegenüberliegenden planaren Seite befinden, also auf der "Rückseite" des plan-konvexen Objektes **(Abb. 1a)**, das mit der Solarzelle verklebt ist. In einer weiteren erfindungsgemäßen Ausführungsform kann sich die wellenlängenselektive Beschichtung auf der Solarzelle befinden, die mit dem facettierten, transparenten Objekt mit konvexer Geometrie verklebt wird **(Abb. 1b) .** In einer weiteren Ausführungsform **(Abb. 1c)** ist eine wellenlängenselektive Folie mit der Solarzelle und mit dem facettierten transparenten Objekt mit konvexer Geometrie mittels zweier Klebstoffschichten verbunden **(Abb. 1c)**.Es sei angemerkt, dass eine Verklebung der einzelnen Teile nicht zwingend notwendig ist.

Erfindungsgemäß kann die wellenlängenselektive Schicht prinzipiell auch auf der facettierten Oberfläche aufgebracht werden; allerdings ist dies aufgrund des möglichen mechanischen Abriebs der Schicht eine der weniger bevorzugten Ausführungsformen. Die phovoltaische Zelle kann ebenso durch Abscheidung beziehungsweise Aufdampfen von Halbleitermaterialien direkt auf das optische Element hergestellt werden, muss also nicht notwendigerweise verklebt werden.

Das Energieelement bietet die Möglichkeit, verschiedene Geräte aus dem Bereich "Wearable Technologies" komplett energieautark zu betreiben oder aber deren Laufzeit abhängig vom einfallenden Licht signifikant zu erhöhen. Durch das permanente Nachladen des Energiespeichers, wird ein gewisses Energieniveau erhalten. Dies erlaubt eine Reduzierung der Ladungsträgerkapazität und folglich des Volumens des Energiespeichers. Daraus ergeben sich Vorteile für das Design des Produkts, beispielsweise eine kompaktere Bauweise, die wiederum zur Kostenreduktion des Produktes beitragen kann. Da das vollständige Laden bzw. Entladen eines konventionellen Energiespeichers durch ein externes Netzteil entfällt, resultiert eine deutlich erhöhte Lebensdauer der derzeit in den Geräten verwendeten Energiespeicher.

Eine Anwendung des Energieelements stellen etwa Finger- bzw. Ohrringe dar, bei denen es als Schmuckstein fungiert und gleichzeitig die nötige Energie für eine integrierte Sensorik samt Sendeeinheit liefert. Derartige Systeme können zur transkutanen, optischen Messung etwa von Laktat, Glucose oder Melatonin im Blut dienen. Das Energieelement erlaubt in Verbindung mit einer speziellen Dünnschichtbatterie und stark miniaturisierter Elektronik erstmals die Integration von Sensorik in ein kompaktes Schmuckstück. So kann beispielsweise ein mit einem Energieelement ausgerüsteter Ring - ohne Batteriewechsel - zur kontinuierlichen Messung bestimmter Körperfunktionen eingesetzt werden.

Auch das partielle Aufladen mobiler Geräte, wie beispielsweise von Mobiltelefonen, Laptops, GPS-Systemen oder Tablets ist durch eine Vielzahl von in Serie bzw. parallel geschalteter Energieelemente möglich. Bei Aufbringung einer Vielzahl solcher Energieelemente auf Kleidung und Accessoires, beispielsweise Taschen, können darin befindliche mobile Geräte induktiv geladen werden. Sogenannte "Energiearmbänder", welche die erfindungsgemäßen Energieelemente enthalten und mit Micro-USB-Steckern versehen sind, können als mobile Ladestationen verwendet werden. Das erfindungsgemäße Energieelement kann auch Energie für sogenannte schaltbare Effekte zur Verfügung stellen, beispielsweise für eine Farbänderung eines Schmucksteins oder beispielsweise die Displayfunktionen einer sogenannten "Smart Watch".

Das Energieelement oder eine Mehrzahl von Energieelementen kann in ein Armband integriert werden, um beispielsweise eine Smart-Watch oder einen Aktivitätssensor (activity tracker) zu versorgen. Eine zuverlässige elektrische Verschaltung der Energieelemente untereinander kann erreicht werden, wenn man die Energieelemente über spezielle Fassungen miteinander verbindet. Eine Energieweiterleitung von den Energieelementen zum Produkt-Teil, das die Energie benötigt, ist beispielsweise über einen speziellen Federsteg (hauptsächlich bei Uhren) oder durch Federkontaktstifte (Pogo-Pins) möglich.

### FACETTIERTES TRANSPARENTES OBJEKT MIT KONVEXER GEOMETRIE

Das facettierte, transparente Objekt mit konvexer Geometrie kann aus einer Vielzahl verschiedenartiger Materialien gefertigt sein, beispielsweise aus transparentem Glas, Kunststoff, transparenter Keramik oder transparenten Edelsteinen oder Halbedelsteinen. Erfindungsgemäß bevorzugt sind facettierte, transparente Objekte mit konvexer Geometrie, die aus Glas oder aus Kunststoff hergestellt sind, da diese am kostengünstigsten sind und am leichtesten mit Facetten versehen werden können. Erfindungsgemäß besonders bevorzugt ist die Verwendung von Glas.

### Glas

Die Erfindung ist bezüglich der Zusammensetzung des Glases prinzipiell nicht limitiert, solange dieses transparent ist. Unter Glas wird eine eingefrorene unterkühlte Flüssigkeit verstanden, die einen amorphen Festkörper bildet. Erfindungsgemäß können sowohl oxidische Gläser als auch Chalkogenidgläser, metallische Gläser oder nicht-metallische Gläser eingesetzt werden. Auch Oxy-Nitrid-Gläser können geeignet sein. Es kann sich um Einkomponenten- (z.B. Quarzglas) oder Zweikomponenten- (z.B. Alkaliboratglas) oder Mehrkomponentengläser (Kalk-Natron-Glas) handeln. Das Glas kann durch Schmelzen, durch Sol-Gel-Prozesse oder auch durch Stoßwellen hergestellt werden. Die Verfahren sind dem Fachmann bekannt. Erfindungsgemäß bevorzugt sind anorganische Gläser, insbesondere oxidische Gläser. Hierzu gehören Silikatgläser, Boratgläser oder Phosphatgläser. Besonders bevorzugt sind bleifreie Gläser.

Für die Herstellung der facettierten transparenten Objekte mit konvexer Geometrie werden silikatische Gläser bevorzugt. Silikatische Gläser haben gemeinsam, dass ihr Netzwerk hauptsächlich aus Siliziumdioxid (SiO₂) gebildet wird. Durch Zugabe weiterer Oxide wie beispielsweise Aluminiumoxid oder verschiedener Alkalioxide entstehen die Alumo- oder Alkali-Silikatgläser. Treten als Hauptnetzwerkbildner eines Glases Phosphorpentoxid oder Bortrioxid auf, spricht man von Phosphat- bzw. Boratgläsern, deren Eigenschaften ebenfalls durch Zugabe weiterer Oxide eingestellt werden können. Auch diese Gläser sind im Sinne der Erfindung einsetzbar. Die genannten Gläser bestehen größtenteils aus Oxiden, weshalb man sie zusammenfassend als oxidische Gläser bezeichnet.

In einer erfindungsgemäß bevorzugten Ausführungsform enthält die Glaszusammensetzung folgende Bestandteile: In einer bevorzugten Ausführungsform enthält die Glaszusammensetzung folgende Komponenten:
(a) etwa 35 bis etwa 85 Gew.-% SiO9₂;
(b) 0 bis etwa 20 Gew.-% K₂O;
(c) 0 bis etwa 20 Gew.-% Na₂O;
(d) 0 bis etwa 5 Gew.-% Li₂O;
(e) 0 bis etwa 13 Gew.-% ZnO;
(f) 0 bis etwa 11 Gew.-% CaO;
(g) 0 bis etwa 7 Gew.-% MgO;
(h) 0 bis etwa 10 Gew.-% BaO;
(i) 0 bis etwa 4 Gew.-% Al₂O₃;
(j) 0 bis etwa 5 Gew.-% ZrO₂;
(k) 0 bis etwa 6 Gew.-% B₂O₃;
(l) 0 bis etwa 3 Gew.-% F;
(m) 0 bis etwa 2,5 Gew.-% Cl.

Alle Mengenangaben sind dabei so zu verstehen, dass sie sich jeweils zu 100 Gew.-% ergänzen. Die Facettierung der Glasobjekte wird durch Schleif- und Poliertechniken erhalten, die dem Fachmann hinlänglich bekannt sind. Erfindungsgemäß geeignet ist beispielsweise ein bleifreies Glas, insbesondere das von der Firma Swarovski für die Chessboard Flat Backs verwendete Glas (Katalog-Nr. 2493), welches eine Transmission von > 95 % im Bereich 380 -1200 nm aufweist.

### Kunststoff

Als weiterer Rohstoff für die Herstellung des facettierten transparenten Objekts mit konvexer Geometrie können transparente Kunststoffe eingesetzt werden. Erfindungsgemäß geeignet sind alle Kunststoffe, die nach dem Härten der Monomere transparent sind; diese sind dem Fachmann hinlänglich bekannt. Hier finden unter anderem folgende Materialien Verwendung:
- Acrylglas (Polymethylmetacrylate, PMMA),
- Polycarbonat (PC),
- Polyvinylchlorid (PVC),
- Polystyrol (PS),
- Polyphenylenether (PPO),
- Polyethylen (PE),
- Poly(n-methylmethacrylimid (PMMI).

Die Vorteile der transparenten Kunststoffe gegenüber Glas liegen insbesondere im geringeren spezifischen Gewicht, das nur rund die Hälfte des Glases beträgt. Auch andere Materialeigenschaften sind gezielt einstellbar. Kunststoffe sind zudem oft einfacher zu bearbeiten als Glas. Nachteilig wirkt sich der im Vergleich zu Glas geringe Elastizitätsmodul und die geringe Oberflächenhärte sowie der massive Festigkeitsabfall bei Temperaturen ab ca. 70°C aus. Ein erfindungsgemäß bevorzugter Kunststoff ist Poly(n-methylmethacrylimid), der beispielsweise von Evonik unter dem Namen Pleximid^{®} TT70 vertrieben wird. Pleximid^{®} TT70 hat einen Brechungsindex von 1,54 und einen Transmissionsgrad von 91 %, gemessen nach ISO 13468-2 unter Verwendung von D65-Normlicht.

### Geometrie

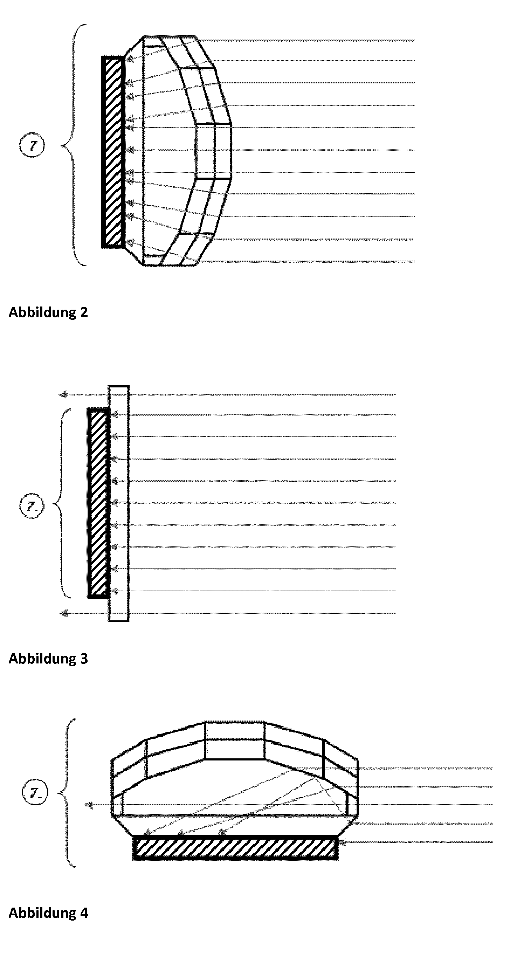

Die geometrische Ausgestaltung des facettierten, transparenten Objektes mit konvexer Geometrie ist prinzipiell nicht limitiert und hängt vorwiegend von Designaspekten ab. Das Objekt ist vorzugsweise quadratisch, rechteckig oder rund. Das facettierte transparente Objekt weist vorzugsweise eine plan-konvexe Geometrie auf (vgl. Abbildungen 2 und 3). Vorzugsweise enthält das Objekt eine Vielzahl von Facetten auf der konvex gekrümmten Seite; insbesondere bevorzugt sind rechteckige und quadratische Facetten, da diese zur Optimierung der Energieausbeute beitragen. Die konvexe Geometrie des Objekts erhöht die Lichtausbeute durch Vergrößerung der gesamten Oberfläche. Während die wellenlängenselektive Schicht (*vide infra*) einen negativen Effekt bezüglich der Lichtausbeute hat, weil ein bestimmter Teil des einfallenden Lichtes reflektiert wird, wird dieser Verlust durch die konvexe Geometrie des Schmucksteins in Kombination mit den Facetten mehr als kompensiert. Insbesondere die konvexe Geometrie des Objektes trägt dazu bei, dass auch die Winkelabhängigkeit der Energieausbeute der Solarzelle entscheidend reduziert wird. Gerade im Hinblick auf tragbare Elektronik, bei der eine Ausrichtung zur Lichtquelle kaum möglich ist, ist eine Reduzierung der Winkelabhängigkeit von sehr großer Bedeutung. Durch die Kombination aus Konvexität und Facettierung wird die Energieausbeute deutlich erhöht. Dies ist in den Abbildungen 2 und 3 schematisch dargestellt. Durch die linsenähnliche Form des Energieelements, die näherungsweise plankonvex ist, werden die Lichtstrahlen auf der Oberfläche des photovoltaischen Elements gebündelt und damit die Energieausbeute der Solarzelle erhöht (Abb. 2a). Gleichzeitig wird, wie Abb. 3a zeigt, die Winkelabhängigkeit im Vergleich zu einer üblicherweise zum Verkapseln von Solarzellen verwendeten dünnen Platte drastisch reduziert. Durch die konvexe Krümmung und die Facettierung und die daraus resultierende zusätzliche Fläche werden die Lichtstrahlen, die auf das Energieelement fallen, zum Lot auf die Solarzelle gebrochen. Durch die Facettierung kommt es zu einer Mehrfachreflexion der Lichtstrahlen (light trapping) und somit zu einer Steigerung der Lichtausbeute.

Die Art der Facettierung hängt eng mit der Geometrie des optischen Elementes zusammen. Prinzipiell ist die geometrische Form der Facetten nicht limitiert. Erfindungsgemäß bevorzugt sind quadratische oder rechteckige Facetten, insbesondere in Kombination mit einem quadratisch oder rechteckig dimensionierten transparenten Objekt mit plankonvexer Geometrie.

### WELLENLÄNGENSELEKTIVE SCHICHT

Die wellenlängenselektive Schicht ermöglicht, dass das Energieelement überhaupt als Schmuckstein verwendet werden kann. Das Energieelement erscheint dadurch brillant. Die wellenlängenselektive Schicht befindet sich bevorzugt zwischen dem transparenten, facettierten Objekt mit konvexer Geometrie und dem photovoltaischen Element. Sie wird erfindungsgemäß bevorzugt auf zwei verschiedene Weisen verwirklicht werden: durch eine wellenlängenselektive Folie oder aber durch eine wellenlängenselektive Beschichtung, die durch PVD, CVD oder nasschemische Verfahren hergestellt wird. Eine wellenlängenselektive Schicht kann aber ebenso durch eine mikrostrukturierte Oberfläche erhalten werden. Dem Fachmann sind die Methoden zur Mikrostrukturierung wohl bekannt.

Durch die Reflexion eines definierten Bereichs (=Filtern) des sichtbaren Spektrums gewinnt das optische Element an Brillanz und erscheint für den Betrachter in einer bestimmten Farbe. Die Brillanz wird durch die Facettierung des konvexen Objektes zusätzlich unterstützt. In einer bevorzugten Ausführungsform der Erfindung reflektiert die wellenlängenselektive Schicht einen Anteil des Lichtes im Bereich von 380 - 850 nm, d.h. im vorwiegend sichtbaren Bereich. Der Anteil des Lichtes, der reflektiert wird, liegt dabei in einem möglichst schmalen Bereich des sichtbaren Spektrums, typischerweise in einem nicht mehr als 50-250nm breiten Intervall. Einerseits ist dieser Anteil ausreichend, um das dekorative Element hinsichtlich der Brillanz als Schmuckstein wahrzunehmen. Andererseits werden Verluste in der Energieausbeute, resultierend aus dem reflektierten Wellenlängenbereich, minimiert. Erfindungsgemäß bevorzugt ist es daher, dass die wellenlängenselektive Schicht das einfallende Licht in einem 50-250nm breiten Reflexionsintervall, welches im Bereich von 380 - 850 nm liegt, wenigstens zu 50% reflektiert. Vorzugsweise ist das Reflexionsintervall 50 - 200 nm breit, besonders bevorzugt 50 - 150 nm. In einer weiteren bevorzugten Ausführungsform hat die wellenlängenselektive Schicht außerhalb des Reflexionsintervalls eine durchschnittliche Transmission von > 60 %, vorzugsweise > 80% im Wellenlängenbereich von 400-1200nm (vgl. Abb. 4a), gemessen bei einem Einfallswinkel der Lichtstrahlen von 0º. Vorzugsweise ist die wellenlängenselektive Schicht auf der Seite des Objektes aufgebracht, die der facettierten Seite gegenüberliegt; alternativ kann sie auch direkt auf das photovoltaische Element aufgebracht werden.

Die photovoltaische Zelle (Solarzelle) kann nur einen Teil des Sonnenspektrums nutzen. Die wellenlängenselektive Schicht, die als Filter wirkt, reflektiert vorzugsweise zusätzlich den Teil des Spektrums, der im IR-Bereich liegt und von der Solarzelle nicht mehr genutzt werden kann und verhindert somit eine zusätzliche Erwärmung der Solarzelle.

Üblicherweise verlieren Solarzellen 0,47% an Energieausbeute pro °C Erwärmung, so dass die korrekte Wahl der Beschichtung von hoher Bedeutung ist. Je kürzer die einfallende Wellenlänge ist desto höher ist die Energie der Photonen (E=h·ν [eV]). Bei Silizium-Solarzellen wird eine Energie von 1.1eV benötigt um ein Elektronen Loch-Paar aus dem p/n Übergang zu schlagen; die überschüssige Energie wird in Wärme umgewandelt. Wenn beispielsweise ein Photon mit 3.1eV entsprechend der Energie bei 400nm auf die Zelle eintrifft, werden 2eV in Wärmeenergie umgewandelt und führen zu einer Reduzierung der Energieausbeute. Daher ist es erfindungsgemäß besonders vorteilhaft den kurzwelligen blauen bzw. grünen Anteil zu reflektieren (Wellenlänge: 380-490nm), da hier am meisten Wärme erzeugt wird. Prinzipiell ist es durch die wellenlängenselektive Schicht möglich, Energieelemente mit den verschiedensten Farben zu generieren. Um die Energieausbeute zu optimieren, ist es jedoch bevorzugt, dass die wellenlängenselektive Schicht einen Anteil aus dem kurzwelligen Bereich des sichtbaren Spektrums reflektiert.

Die wellenlängenselektive Schicht zeigt eine winkelabhängige Reflexion (Abb. 4a und 4b). Das Reflexionsintervall verschiebt sich je nach Einfallswinkel des Lichtes auf die Facetten. In Abhängigkeit von der Lage der Facetten werden unterschiedliche Farbanteile reflektiert und es entsteht ein annähernd irisierender Effekt, d.h. eine graduelle Farbveränderung von Facette zu Facette, die mit einer plan-konvexen Linse ohne Facetten nicht erreicht werden kann.

Die wellenlängenselektive Schicht ist vorzugsweise zumindest partiell für UV-Licht durchlässig, um auch eine Verklebung der einzelnen Komponenten des Energieelementes mit UV-härtenden Klebstoffen zu ermöglichen.

### Wellenlängenselektive Folien

Wellenlängenselektive Folien werden unter dem Begriff "Radiant Light Film" im Handel vertrieben. Hierbei handelt es sich um mehrschichtige polymere Filme, die auf andere Materialien appliziert werden können. Diese Lichtfolien sind Bragg-Spiegel und reflektieren einen hohen Anteil des sichtbaren Lichts und erzeugen brillante farbige Effekte. Eine reliefartige Mikrostrukturierung im Bereich von mehreren hundert Nanometern reflektiert die verschiedenen Wellenlängen des Lichtes Licht und es kommt zu Interferenzerscheinungen, wobei sich die Farben je nach Blickwinkel ändern.

Erfindungsgemäß besonders bevorzugte Folien bestehen aus mehrschichtigen polymeren Filmen, deren äußere Schicht ein Polyester ist. Solche Filme werden beispielsweise von der Firma 3M unter dem Namen Radiant Color Film CM 500 und CM 590 vertrieben. Die Filme haben ein Reflexionsintervall von 590 - 740 bzw. 500 - 700 nm.

Die wellenlängenselektive Folie wird vorzugsweise mittels eines Klebstoff mit der photovoltaischen Zelle und dem facettierten transparenten Objekt verbunden. Der Klebstoff sollte ebenfalls transparent sein. In einer bevorzugten Ausführungsform weicht der Brechungsindex des Klebstoffs weniger als ± 20 % von dem Brechungsindex des facettierten, transparenten Körpers mit konvexer Geometrie ab. In einer besonders bevorzugten Ausführungsform ist die Abweichung < 10 %, ganz besonders bevorzugt < 5 %. Nur auf diese Weise wird gewährleistet, dass Reflexionsverluste aufgrund der unterschiedlichen Brechungsindizes minimiert werden können. Die Brechungsindices können auch durch Aufrauhung der jeweiligen Grenzschichten aneinander angepasst werden (Mottenaugeneffekt). Sogenannte "Mottenaugenoberflächen" bestehen aus feinen Noppenstrukturen, die das Brechungsverhalten des Lichtes nicht schlagartig, sondern idealerweise kontinuierlich verändern. Dadurch werden die scharfen Grenzen zwischen den unterschiedlichen Brechzahlen beseitigt, so dass der Übergang fast fließend erfolgt und das Licht ungehindert hindurch dringen kann. Die Strukturgrößen, die dafür erforderlich sind, müssen kleiner als 300 nm sein. Durch Mottenaugeneffekte wird sichergestellt, dass die Reflexion an den Grenzschichten minimiert wird und somit eine höhere Lichtausbeute beim Durchgang durch die Grenzschichten erzielt wird.

Klebstoffe, die mittel UV ausgehärtet werden können, sind erfindungsgemäß bevorzugt. Dem Fachmann sind sowohl die UV-härtenden Klebstoffe wohl bekannt als auch die Verfahren zur Bestimmung des Brechungsindex. Erfindungsgemäß besonders bevorzugt ist die Verwendung von Acrylat-Klebstoffen, insbesondere von modifizierten Urethanacrylat-Klebstoffen. Diese werden von zahlreichen Firmen vertrieben, beispielsweise von Delo unter der Bezeichnung Delo-Photobond^{®} PB 437, ein Klebstoff, der durch UV-Licht im Bereich von 320-42 nm ausgehärtet werden kann.

### Wellenlängenselektive Beschichtung

Die Beschichtungsmaterialien sind dem Fachmann wohl bekannt. In einer bevorzugten Ausführungsform der Erfindung enthalten die wellenlängenselektiven Beschichtungen wenigstens ein Metall und/oder eine Metallverbindung, wie beispielsweise Metalloxide, Metallnitride, Metallfluoride, Metallcarbide oder eine beliebige Kombination dieser Verbindungen in beliebiger Reihenfolge, die mittels einer der gängigen Beschichtungsverfahren auf die facettierten Schmucksteine aufgebracht werden. Es können auch aufeinanderfolgende Schichten verschiedener Metalle oder Metallverbindungen aufgebracht werden. Die Verfahren zur Herstellung von Beschichtungen sowie die Beschichtungen selbst sind dem Fachmann hinlänglich bekannt. Hierzu zählen unter anderem PVD (physical vapour deposition), CVD (chemical vapour deposition), Lackieren sowie nasschemische Verfahren gemäß Stand der Technik. Erfindungsgemäß bevorzugt sind PVD-Verfahren.

Bei den PVD-Methoden handelt es sich um eine Gruppe von vakuumbasierten Beschichtungsverfahren bzw. Dünnschichttechnologien, die dem Fachmann hinlänglich bekannt sind und insbesondere zur Beschichtung von Glas und Kunststoff in der Optik- und der Schmuckindustrie eingesetzt werden. Im PVD-Prozess wird das Beschichtungsmaterial in die Gasphase überführt. Das gasförmige Material wird anschließend zum zu beschichtenden Substrat geführt, wo es kondensiert und die Zielschicht bildet. Mit einigen dieser PVD-Verfahren (Magnetronsputtern, Laserstrahlverdampfen, thermische Bedampfung, etc.) können sehr niedrige Prozesstemperaturen verwirklicht werden. Eine Vielzahl von Metallen kann auf diese Weise in sehr reiner Form in dünnen Schichten abgeschieden werden. Führt man den Prozess in Gegenwart von Reaktivgasen wie Sauerstoff durch, so lassen sich auch Metalloxide abscheiden. Ein erfindungsgemäß bevorzugtes Verfahren ist ein Beschichtungsprozess mittels Sputtern. Ein typisches Schichtsystem kann je nach Anforderung an Funktion und optische Erscheinung aus nur einer, aber auch aus einer Vielzahl von Schichten bestehen. In der Praxis beschränkt man sich meist auf Schichtanzahlen zwischen 1 und 25. Die typische Schichtdicke variiert zwischen 5 und 800 nm. Als Beschichtungsmaterialien sind erfindungsgemäß insbesondere Cr, Cr₂O₃, Ni, NiCr, Fe, Fe₂O₃, Al, Al₂O₃, Au, SiOₓ, Mn, Si, Si₃N₄, TiOₓ, Cu, Ag, Ti,CeF₃, MgF₂, Nb₂O₅, Ta₂O₅, SnO₂, ZnO₂, MgO, CeO₂, WO₃, Pr₂O₃, Y₂O₃; BaF₂, CaF₂, LaF₃, NdF₃, YF₃; ZrO₂, HfO₂, ZnS, Oxinitride von Al, Si, sowie SnZnO geeignet.

Um eine wellenlängenselektive Beschichtung zu erhalten, können beispielweise absorbierende Materialien verwendet werden, die aufgrund ihres Absorptionsverhaltens wellenlängenselektiv nur gewisse Anteile des sichtbaren Lichtes transmittieren bzw. reflektieren und dadurch farbig sind. Erfindungsgemäß bevorzugt geeignet sind Schichtsysteme, die aus dielektrischen Materialien aufgebaut sind und aufgrund von Interferenzerscheinungen nur gewisse Anteile des sichtbaren Lichtes transmittieren bzw. reflektieren und dadurch farbig wirken, beispielsweise eine Vielfach-Abfolge von TiO₂ und SiO₂. Eine erfindungsgemäß besonders bevorzugte wellenlängenselektive Beschichtung besteht aus einer alternierenden Abfolge von TiO₂ und SiO₂ in zwölf Schichten und Schichtdicken, die zwischen ca. 20-145 nm variieren. Erfindungsgemäß bevorzugt sind sogenannte Bandsperrfilter mit den Kantenlagen 380 und 480 nm, d.h. dass im Bereich 380-480 nm der größte Anteil des Lichtes reflektiert wird (=Reflexionsintervall; vgl. Abbildung 4). Zur Herstellung von Bandsperrfiltern anderer Kantenlagen werden Schichtanzahl und Schichtdicke variiert. Für die PVD-Schichterzeugung steht eine Vielzahl handelsüblicher Maschinen zur Verfügung, beispielsweise das Modell BAK1101 der Firma Evatec.

### PHOTOVOLTAISCHES ELEMENT

Das photovoltaische Element (Solarzelle) ist ein elektrisches Bauelement, das kurzwellige Strahlungsenergie, in der Regel Sonnenlicht, direkt in elektrische Energie umwandelt. Welche Art von Solarzelle einsetzbar ist, hängt von der erforderlichen Energieversorgung sowie dem speziellen Applikationszweck ab. Für den erfindungsgemäßen Applikationszweck eignen sich insbesondere anorganische Solarzellen. Sie werden aus Halbleitermaterialien, am häufigsten aus Silicium, gefertigt. Daneben finden unter anderem auch Cadmiumtellurid, Kupfer-Indium-Gallium-Diselenid und Galliumarsenid Anwendung. Bei sogenannten Tandem-Solarzellen werden Schichten unterschiedlicher Halbleiter verwendet, beispielsweise Indiumgalliumarsenid in Kombination mit Indiumgalliumphosphid.

Neben dem Material ist die Bauweise der Solarzelle von Bedeutung. Stapeltechniken mit Materialkombinationen werden beispielsweise verwendet, um den Wirkungsgrad der Gesamtanordnung zu erhöhen. Die Materialien werden so gewählt, dass das einfallende Sonnenspektrum maximal ausgenutzt wird. Während der theoretisch erreichbare Wirkungsgrad bei ca. 43% liegt, werden in der Realität in Standard-Solarzellen nur ca. 15 bis 20 % erreicht. Verluste entstehen durch Rekombination der Ladungsträger und einhergehender Wärmeerzeugung, durch Reflexion sowie aufgrund des Serienwiderstandes. Die elektrische Spannung bei maximaler Leistung (Maximum Power Point, Leistungsanpassung) liegt bei den gebräuchlichsten Zellen (kristalline Siliziumzellen) bei etwa 0,5 V.

Die Struktur von Solarzellen wurde in den letzten Jahren optimiert, so dass möglichst viel Licht absorbiert wird und in der aktiven Schicht freie Ladungsträger erzeugt werden. Dazu wird eine Antireflexionsschicht auf die Oberseite der Solarzelle aufgetragen während die Rückseite verspiegelt wird. Die Antireflexionsschicht sorgt für die typisch bläuliche bis schwarze Farbe von Solarzellen. Die Antireflexionsschicht wird häufig aus Siliciumnitrid, Siliciumdioxid und Titandioxid hergestellt. Über die Schichtdicke der Antireflexbeschichtung wird auch die Farbe bestimmt (Interferenzfarbe). Eine gleichmäßige Schichtdicke ist wichtig, da bereits Schwankungen im Nanometer-Bereich den Reflexionsgrad erhöhen. Blaue Reflexion ergibt sich aus der Einstellung der Antireflexschicht auf den roten Teil des Spektrums - der bevorzugten Absorptionswellenlänge des Siliciums. Siliciumnitrid und Siliciumdioxid als Antireflexschicht-Materialien fungieren zusätzlich als Passivierungsschicht, die die Rekombination von Ladungsträgern an der Oberfläche herabsetzt, so dass mehr Ladungsträger zur Stromerzeugung zur Verfügung stehen. Eine weitere Effizienzsteigerung erreicht man, wenn man die frontseitigen Kontaktfinger auf der Rückseite der Solarzelle anbringt. Hierdurch wird die Abschattung auf der Frontseite vermieden, die eine geringere aktive Fläche und folglich eine geringere Lichtausbeute zur Folge hat, da bis zu 10% der Oberfläche von den Metallkontakten bedeckt ist. Rückseitige Kontaktfinger sind darüber hinaus leichter und verlustärmer elektrisch kontaktierbar als frontseitige Kontaktfinger. Erfindungsgemäß bevorzugt sind rückseitenkontaktierte Solarzellen. Diese sogenannten IBC-Zellen (Interdigitated Back Contact Cells) werden beispielsweise von der Firma SunPower vermarktet. Erfindungsgemäß geeignet sind insbesondere Solarzellen aus monokristallinem Silicium und einer Antireflexbeschichtung aus Siliciumnitrid; vorzugsweise weisen die Solarzellen eine Effizienz > 20 % auf. Erfindungsgemäß besonders geeignet ist die Sunpower^{®} C60-Solarzelle aus monokristallinem Silizium, die sich durch eine Effizienz von ca. 22.5 % auszeichnet. Die Antireflexbeschichtung aus Siliziumnitrid (Si₃N₄) hat typischerweise einen Brechungsindex von 1,9-2,5. Zur Effizienzsteigerung der Solarzellen tragen unter anderem eine Rückseitenkontaktierung, eine Rückseitenverspiegelung, eine Passivierungsschicht aus Siliciumdioxid sowie die Verwendung von n-dotiertem Silicium bei.

Die erfindungsgemäß einsetzbare Größe/Fläche der Solarzelle und des erfindungsgemäßen Energieelementes ist von der Anwendung und von der Bestrahlungsstärke abhängig. Bei einer Fläche von 1cm² und einer Zelleneffizienz von ca. 20% lassen sich bei einer Bestrahlungsstärke von 100 mW/cm² in direktem Sonnenlicht innerhalb einer Stunde theoretisch bis zu 20 mWh an Energie sammeln. In der Praxis wird dieser Wert aufgrund elektrischer Verluste beim Laden des Energiespeichers - sowie der Tatsache, dass eine durchschnittliche Bestrahlungsstärke von ca. 100 mW/cm² bzw. 1000 W/m² in Mitteleuropa nicht häufig erreicht wird - etwas niedriger ausfallen. Bezogen auf einen am Markt erhältlichen "Activity Tracker", der eine durchschnittliche Entladung von ca. 3 mWh/Tag aufweist, würde bei einer Fläche von 1 cm² Solarzelle eine Bestrahlungsdauer von einer Stunde pro Woche in direktem Sonnenlicht ausreichen. Aufgrund der guten Performance der IBC-Solarzellen auch unter nicht-optimalen Lichtbedingungen ist auch eine Verwendung in Innenräumen ausreichend, um einer Entladung der tragbaren elektronischen Geräte entgegenzuwirken. Im Vergleich zu direktem Sonnenlicht im Freien, ist die Bestrahlungsstärke in Räumen um den Faktor 100-200 geringer. Die anfangs genannten Sensoren zur Überwachung von Körperfunktionen zeigen eine durchschnittliche Entladung von ca. 1 bis 5 mWh/Tag. Auch hier ist eine Energieversorgung über das erfindungsgemäße Energieelement möglich, beispielsweise durch Integration des Energieelementes oder einer Vielzahl solcher Elemente in Schmuck-Designs.

In einer bevorzugten Ausführungsform der Erfindung ist das photovoltaische Element mit elektrischen Kontakten versehen, um die generierten elektrischen Ladungsträger in Form von Strom abzuleiten. Dabei werden die rückseitigen elektrischen Kontakte der Solarzelle über eine Leiterplatte kontaktiert und zu einem positiven und einem negativen Kontakt zusammengeführt.

Die Erfindung wird nachfolgend an Hand von Beispielen und Abbildungen näher illustriert ohne darauf beschränkt zu sein. Die Abbildungen zeigen folgende Gegenstände:
- **Abb. 1:**: Aufbau eines Energieelements
- **Abb. 2:**: Bündelung von Lichtstrahlen auf der Solarzelle bei plankonvexem optischem Element mit Facettierung
- **Abb. 3:**: Strahlengang bei planarer Abdeckung der Solarzelle
- **Abb.4:**: Brechung von seitlich einfallenden Lichtstrahlen bei plankonvexem optischem Element mit Facettierung
- **Abb. 5:**: Strahlengang bei seitlich einfallenden Lichtstrahlen bei planarer Abdeckung der Solarzelle
- **Abb.6:**: Spektrum der wellenlängenselektiven Filter-Beschichtung gemäß Tabelle 1 T = Transmission; R = Reflexion
- **Abb. 7:**: Winkelabhängigkeit der Reflexion der wellenlängenselektiven Filter-Beschichtung R = Reflexion.
- **Abb. 8:**: Geometrie des optischen Elements; perspektivisch

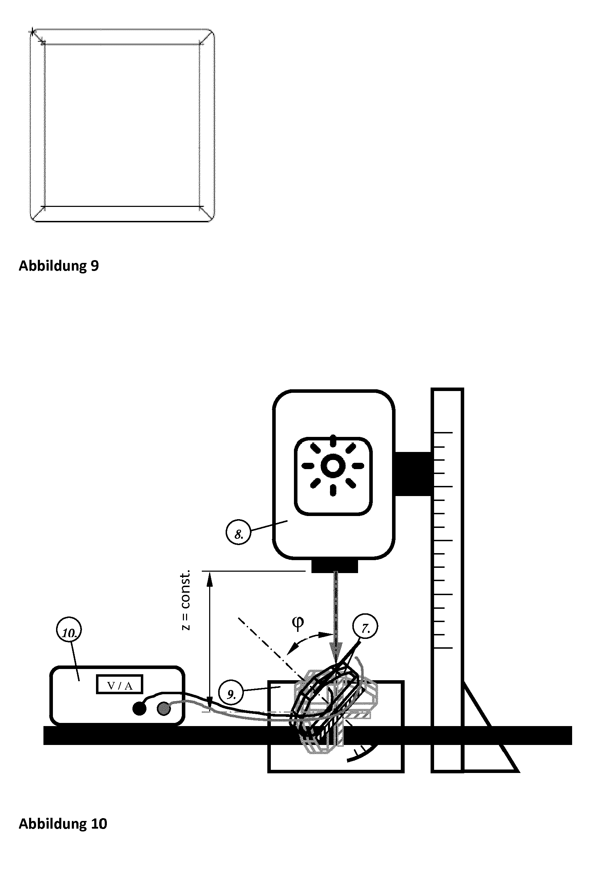

- **Abb. 9:**: Grundfläche des optischen Elements; 45º Phase an der Grundfläche
- **Abb.10:**: Messaufbau - schematisch

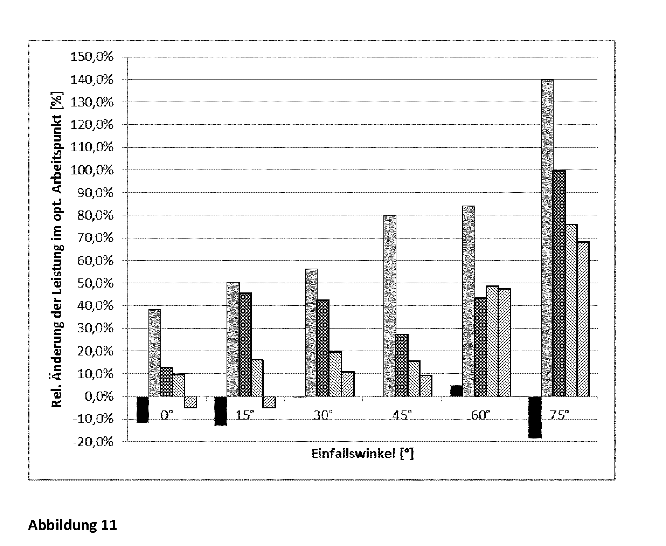

- **Abb. 11:**: Relative Änderung der Leistung im optimalen Arbeitspunkt in Abhängigkeit vom Einfallswinkel der Strahlung.

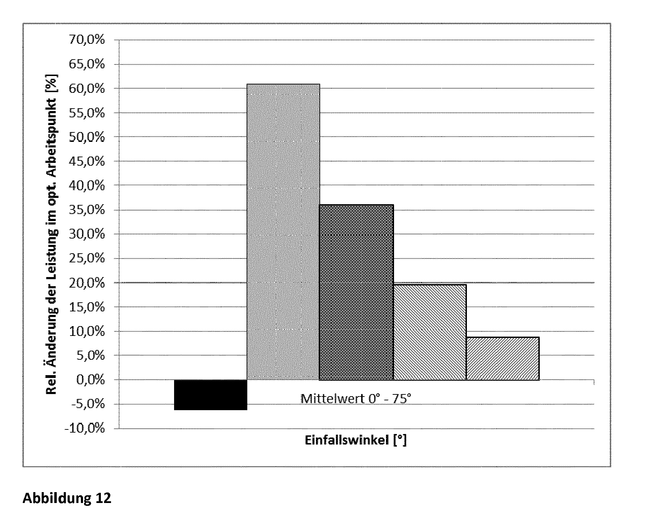

- **Abb. 12:**: Relative Änderung der Leistung im optimalen Arbeitspunkt nach Applikation optischer Elemente gemittelt über die Einfallswinkel 0-75°.

### GEWERBLICHE ANWENDBARKEIT

Weitere Gegenstände der Erfindung betreffen: zum einen die Verwendung des Energieelements gemäß vorliegender Erfindung als Energiequelle, insbesondere in tragbaren elektronischen Geräten, sowie Gegenstände, insbesondere Schmuckteile wie etwa Ringe, Ketten, Armbänder und dergleichen, enthaltend wenigstens ein Energieelement gemäß vorliegender Erfindung

### MATERIALIEN

Es wurden verschiedene Energieelemente untersucht, die sich bezüglich Material und Geometrie unterscheiden. Die Energieelemente wurden aus Solarzellen und optischen Elementen aufgebaut. Die erfindungsgemäßen Beispiele wurden zusätzlich mit einer wellenlängenselektiven Schicht versehen.

**Solarzellen.** Es wurden Solarzellen des Typs Sunpower C60 (10 mm x 10 mm) verwendet.

**Optische Elemente - mit und ohne Beschichtung.** Die optischen Elemente aus Glas wurden durch die dem Fachmann bekannten Verfahren aus käuflich erhältlichen "Chessboard Flat Back" 2493-Elementen (30 mm x 30 mm) der Firma Swarovski gefertigt.

Die optischen Elemente aus Pleximid^{®} TT70 wurden mittels Kunststoffspritzgussverfahren in einer dafür vorgefertigten Form hergestellt. Hierfür wurde eine Spritzgießmaschine der Firma Engel vom Typ e-victory 80/50 verwendet; Temperatur Massezylinder: 210°C steigend bis 280°C, Düse 280°C; Temperatur Werkzeug: 180° DS, 140° AS; Spritzdruckgrenze: 1200 bar; Spritzgeschwindigkeit: ca. 15 cm³/sec; Prägedruck: ca. 800 bar; keine Lösungsmittel.

**Geometrie.** Die optischen Elemente gemäß V2-V5 sind facettierte Körper mit 12mm Kantenlänge und quadratischer Grundfläche mit leicht abgerundeten Ecken. An der Grundfläche ist eine Abschrägung im Winkel von 45º (Phase) angebracht, so dass die tatsächlich verbleibende Grundfläche 10mm x 10mm beträgt. Der facettierte obere Teil mit 25 Facetten in quadratischer Anordnung bildet ein Kugelsegment. Die Gesamthöhe des Körpers beträgt 5,56mm, an den Ecken beträgt die Höhe 1,93mm.

### BESCHREIBUNG DER BEISPIELE UND VERGLEICHSBEISPIELE

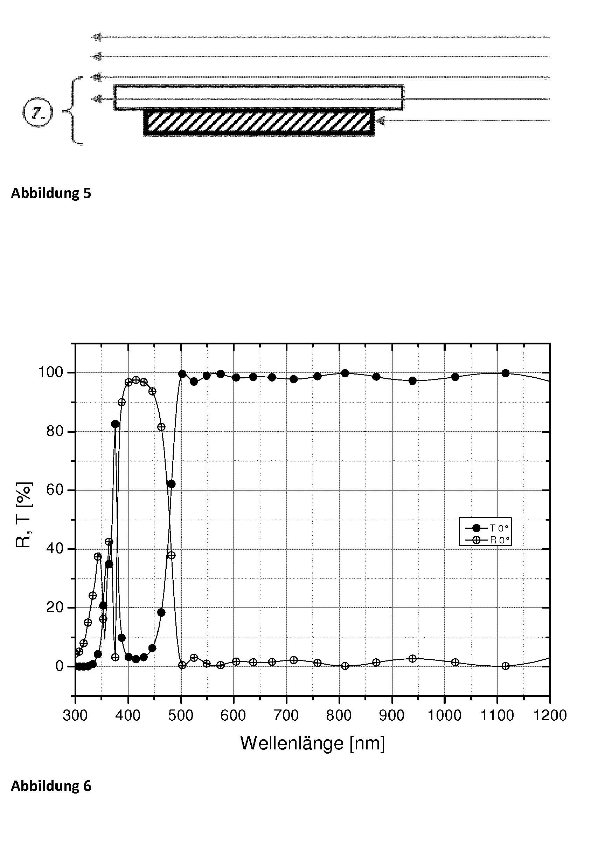

VERGLEICHSBEISPIEL V1: 12mm x 12mm Glasplatte der Stärke 0,5 mm; Brechungsindex n = 1,52
VERGLEICHSBEISPIEL V2: Ein aus Glas gefertigtes optisches Element gemäß Abb. 5; Dimensionen wie nachstehend beschrieben (Geometrie); ohne wellenlängenselektive Beschichtung
BEISPIEL 1: Ein aus Glas gefertigtes optisches Element gemäß Abb. 5; Dimensionen wie nachstehend beschrieben (Geometrie); mit der nachfolgend beschriebenen wellenlängenselektiven Beschichtung
VERGLEICHSBEISPIEL V3: Ein aus Pleximid^{®} TT70 gefertigtes optisches Element gemäß Abb. 5 mit n = 1,54; Dimensionen wie nachstehend beschrieben (Geometrie); ohne wellenlängenselektive Beschichtung (Vergleichsbeispiel)
BEISPIEL 2: Ein aus Pleximid^{®} TT70 gefertigtes optisches Element laut Abb. 5; Dimensionen wie nachstehend beschrieben (Geometrie); mit der nachfolgend beschriebenen wellenlängenselektiven Beschichtung

### WELLENLÄNGENSELEKTIVE SCHICHT

Die optischen Elemente gemäß V3 und V5 wurden in einer PVD-Anlage (*vide supra*) beschichtet. Der Aufbau der wellenlängenselektiven Beschichtung ist in der **folgenden Tabelle 1** wiedergegeben:

**Tabelle 1**

| Schichtaufbau der wellenlängenselektiven Beschichtung | | |
|---|---|---|
| **N** | **Material** | **Physikalische Schichtdicke [nm]** |
| 1 | TiO₂ | 23,9 |
| 2 | SiO₂ | 43,2 |
| 3 | TiO₂ | 64,8 |
| 4 | SiO₂ | 28,7 |
| 5 | TiO₂ | 61,5 |
| 6 | SiO₂ | 33,7 |
| 7 | TiO₂ | 57,7 |
| 8 | SiO₂ | 37,5 |
| 9 | TiO₂ | 66,1 |
| 10 | SiO₂ | 30,5 |
| 11 | TiO₂ | 42,6 |
| 12 | SiO₂ | 141,4 |

### MESSAUFBAU UND MESSUNGEN

Ziel der Messungen war es, den Einfluss des optischen Elementes und der Beschichtung auf die Energieausbeute der Solarzelle in Abhängigkeit vom Einfallswinkel der Lichtstrahlen zu untersuchen.

**Energieelemente:** Es wurden fünf verschiedene Energieelemente untersucht, die aus den optischen Elementen gemäß den Beispielen und Vergleichsbeispielen und den Solarzellen des Typs Sunpower C60 (10 mm x 10 mm) aufgebaut waren.

Die Messungen wurden mit einem Oriel Instruments LED-Sonnensimulator Verasol-2 (Klasse AAA) unter Verwendung eines Keithley 2602A Sourcemeters und einer Linos Drehfassung inklusive entsprechender Halterung durchgeführt.

Eine schematische Darstellung des Messaufbaus zeigt **Abbildung 6****.** Die Bezugszeichen stehen hierbei für:
(7) Energieelement;
(8) Sonnensimulator;
(9) Drehfassung;
(10) Sourcemeter.

Mit Hilfe eines nach Klasse AAA (spektrale Übereinstimmung, räumliche Gleichförmigkeit, Zeitstabilität) zertifizierten Sonnensimulators (8) wurde für die komplette Versuchsreihe eine konstante Bestrahlungsstärke von 1000W/m² gewählt. Der Einfallswinkel der Strahlung der Lichtquelle auf die zu vermessenden Proben gemäß V1 bis V5 wurde dabei mit Hilfe einer Drehfassung (9) variiert, auf der die Proben positioniert wurden. Der Abstand z zwischen dem Mittelpunkt der Solarzelle und dem Sonnensimulator wurde konstant gehalten (vgl. **Abb. 6**).

Mittels Sourcemeter (10) wurde jeweils die Strom-Spannungskennlinie der Solarzellen bei einer Bestrahlungsstärke von 1000W/m² gemessen und daraus die Leistung im optimalen Arbeitspunkt (Maximum Power Point) ermittelt.

Jede der fünf Solarzellen wurde zuerst ohne optische Elemente vermessen. Der Einfallswinkel der Lichtstrahlen wurde dabei in 15°-Schritten von 0° bis 75° variiert (vgl. Abb. 6). Anschließend wurde auf jede Solarzelle jeweils ein optisches Element (*vide supra*) mit wellenlängenselektiver Beschichtung **(Tabelle 1)** mittels eines UV-Klebstoffs mit einem Brechungsindex n=1,461 appliziert und die komplette Messreihe wiederholt. Jede Einzelmessung wurde dreimal wiederholt; daraus wurde der arithmetische Mittelwert gebildet und die relative Standardabweichung (Standardabweichung/ Mittelwert) berechnet; die Ergebnisse sind in **Tabelle 2** zusammengefasst.

**Tabelle 2**

| Relative Änderung der Leistung der Solarzelle im optimalen Arbeitspunkt (Maximum Power Point) in Abhängigkeit vom Einfallswinkel der Lichtstrahlen für die oben beschriebenen Versuchsanordnungen. | | | | | |
|---|---|---|---|---|---|
| **Einfallswinkel** | **V1** | **V2** | **1** | **V3** | **2** |
| 0° | -11,5% | 38,2% | 12,7% | 9,4% | -5,1% |
| 15° | -12,8% | 50,3% | 45,5% | 16,0% | -5,0% |
| 30° | -0,6% | 56,4% | 42,5% | 19,4% | 10,9% |
| 45° | 0,1% | 79,9% | 27,2% | 15,5% | 9,4% |
| 60° | 4,7% | 84,0% | 43,3% | 48,8% | 47,4% |
| 75° | -18,5% | 140,0% | 99,5% | 76,1% | 68,1% |
| Mittelwert 0-75° | -6,4% | 60,8% | 35,9% | 19,7% | 8,7% |

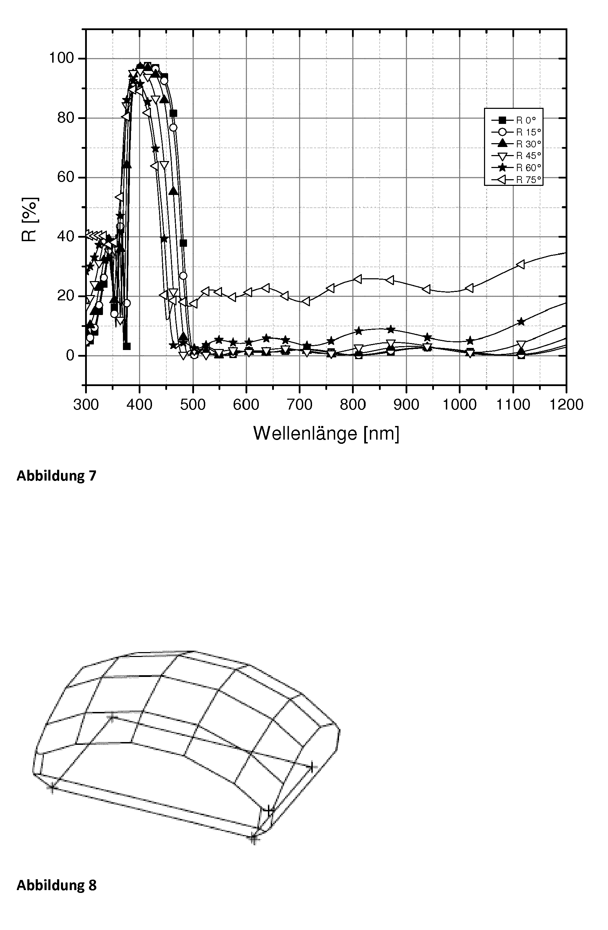

Eine graphische Auswertung der in **Tabelle 2** erhaltenen Ergebnisse ist in den Abbildungen 7a und 7b zu finden. Hierbei bedeutet ■ Schwarz: V1; ▩ Grau: V2; ▦ Doppelschraffur: 1; ▧ Schraffur von links oben nach rechts unten: V3; ▨ Schraffur von links unten nach rechts oben: V5;

### DISKUSSION DER ERGEBNISSE

Die Leistungsverluste bei Beispiel V1 resultieren einerseits aus Verlusten aufgrund unterschiedlicher Brechungsindizes beim Übergang Glas/UV-Klebstoff und UV-Klebstoff/Solarzelle. Andererseits geht ein Teil der Lichtstrahlen beim Auftreffen auf die Glasplatte durch Reflexion verloren. Grundsätzlich treten beide Arten des Leistungsverlusts bei allen optischen Elementen auf, jedoch sind sie bei planaren Glasplatten am größten.

Die Steigerung der Leistung der Solarzelle im optimalen Arbeitspunkt (Maximum Power Point) ist vorwiegend durch geometrische Effekte bedingt, wie in **Abb. 2 und Abb. 3** dargestellt. Vor allem bei zunehmendem Einfallswinkel der Lichtstrahlen lässt sich die Relevanz der konvexen Geometrie mit Facettierung erkennen. Die Leistung des Energieelementes steigt stark an (Beispiele V2 und V3). Für Glas fällt diese noch deutlicher aus als für die aus Pleximid^{®} TT70 gefertigten optischen Elemente.

Durch die wellenlängenselektive Beschichtung (Beispiel 1 und 2) ergeben sich erwartungsgemäß Verluste beim Energieertrag im Vergleich zu V2 und V3 aufgrund der Reflexion eines Teils des sichtbaren Spektrums. Jedoch können diese, wie aus **Tabelle 2** ersichtlich, durch die konvexe Geometrie in Kombination mit der Facettierung mehr als kompensiert werden.

Die Unterschiede zwischen den optischen Elementen aus Glas und Pleximid^{®} TT70 resultieren aus dem deutlich besseren Transmissionsverhalten von Glas sowie der herstellungsbedingten besseren Oberflächenqualität der Glasproben.

## Patentansprüche

1. Energieelement, enthaltend
(a) ein facettiertes, transparentes Objekt mit konvexer Geometrie,
(b) eine wellenlängenselektive Schicht und
(c) eine photovoltaische Zelle.

2. Energieelement nach Anspruch 1, **dadurch gekennzeichnet, dass** das facettierte, transparente Objekt aus Glas oder aus Kunststoff gefertigt ist.

3. Energieelement nach Anspruch 1, **dadurch gekennzeichnet, dass** das facettierte, transparente Objekt eine plan-konvexe Geometrie aufweist.

4. Energieelement nach Anspruch 1, **dadurch gekennzeichnet, dass** die wellenlängenselektive Schicht ausgewählt ist aus einer wellenlängenselektiven Beschichtung oder einer wellenlängenselektive Folie.

5. Energieelement nach Anspruch 4, **dadurch gekennzeichnet, dass** die wellenlängenselektive Beschichtung wenigstens Metall und/oder eine Metallverbindungen enthält.

6. Energieelement nach mindestens einem der Ansprüche 1 bis 5, **dadurch gekennzeichnet, dass** die wellenlängenselektive Schicht einen Anteil des Lichtes im Bereich von 380 - 850 nm reflektiert.

7. Energieelement nach mindestens einem der Ansprüche 1 bis 6, **dadurch gekennzeichnet, dass** die wellenlängenselektive Schicht das einfallende Licht in einem 50-250nm breiten Reflexionsintervall, welches im Bereich von 380 - 850 nm liegt, wenigstens zu 50% reflektiert.

8. Energieelement nach Anspruch 7, **dadurch gekennzeichnet, dass** die wellenlängenselektive Schicht außerhalb des Reflektionsintervalls eine durchschnittliche Transmission > 80% im Bereich von 400-1200nm aufweist, gemessen bei einem Einfallswinkel der Lichtstrahlen von 0º.

9. Energieelement nach mindestens einem der Ansprüche 1 bis 8, **dadurch gekennzeichnet, dass** die wellenlängenselektive Schicht auf der
(a) der facettierten Seite gegenüberliegenden Seite aufgebracht ist oder
(b) auf der photovoltaischen Zelle aufgebracht ist.

10. Energieelement nach mindestens einem der Ansprüche 1 bis 9, **dadurch gekennzeichnet, dass** die wellenlängenselektive Beschichtung wenigstens eine Verbindung umfastt, die ausgewählt ist aus der Gruppe, die gebildet wird von Cr, Cr₂O₃, Ni, NiCr, Fe, Fe₂O₃, Al, Al₂O₃, Au, SiOₓ, Mn, Si, Si₃N₄, TiOₓ, Cu, Ag, Ti,CeF₃, MgF₂, Nb₂O₅, Ta₂O₅, SnO₂, ZnO₂, MgO, CeO₂, WO₃, Pr₂O₃, Y₂O₃; BaF₂, CaF₂, LaF₃, NdF₃, YF₃; ZrO₂, HfO₂, ZnS, Oxinitride von Al, Si, und SnZnO oder eine beliebige Kombination dieser Verbindungen in beliebiger Schichtabfolge.

11. Energieelement nach mindestens einem der Ansprüche 1 bis 10, **dadurch gekennzeichnet, dass** die photovoltaische Zelle eine rückseitenkontaktierte Solarzelle ist.

12. Energieelement nach mindestens einem der Ansprüche 1 bis 11, **dadurch gekennzeichnet, dass** die Komponenten (a), (b) und (c) des Energieelementes mittels eines Klebstoffs verbunden sind.

13. Energieelement nach Anspruch 12, **dadurch gekennzeichnet, dass** der Brechungsindex des Klebstoffs weniger als ± 20 % von dem Brechungsindex des facettierten, transparenten Körpers mit konvexer Geometrie abweicht.

14. Verwendung des Energieelements gemäß mindestens einem der Ansprüche 1 bis 3 als Energiequelle, insbesondere in tragbaren elektronischen Geräten.

15. Gegenstand enthaltend wenigstens ein Energieelement gemäß mindestens einem der Ansprüche 1 bis 13.
